# EUROPEAN PATENT SPECIFICATION

(11) **EP 1 527 770 B1**
(45) Date of publication and mention of the grant of the patent: **17.12.2008**
(21) Application number: 05002381.1
(22) Date of filing: 04.02.2005
(51) Int. Cl.: A61K 8/02, A61K 8/67, A61Q 19/08

(54) **A cosmetic mask composition**
Kosmetische Maskenzusammensetzung
composition cosmétique pour former une masque de beauté

(30) Priority: 08.04.2004 US 822056
(43) Date of publication of application: 04.05.2005
(73) Proprietor: Castiglioni, Mauro, 20030 Lentate Sul Seveso ( Milano) (IT); Forte, Riccardo, 22070 Cassina Rizzardi ( Como ) (IT)
(72) Inventor: Castiglioni, Mauro, 20030 Lentate Sul Seveso ( Milano) (IT); Forte, Riccardo, 22070 Cassina Rizzardi ( Como ) (IT)
(74) Representative: Long, Giorgio

(56) References cited:
- EP-A- 0 443 413
- EP-A- 1 344 528
- WO-A-82/04393
- WO-A-02/062132
- US-A- 4 743 441

## Description

### Field of the invention

The present invention relates to a cosmetic composition, particularly a self-curing mask, to be used as an "anti-ageing" treatment.

### Background of the art

Cutaneous ageing is a widely studied phenomenon and constitutes a particularly felt problem throughout the population, particularly female, both in relation to age and to the ever more frequent exposure to solar rays. Photo-induced cutaneous ageing is caused by the fact that ultraviolet rays induce errors in the transcription of some epithelial cell genes, which are thus not able to carry out all their normal functions. In practice, senescent keratinocyte cells stop growing and do not enter into the cell cycle S-phase, independently of any mitotic stimulation, and some genes required for the cell cycle are inhibited. However, the senescent keratinocytes maintain normal metabolic activity as well as the possibility of synthesising protein or RNA for a certain period of time.

Retinoic acid, or tretinoine, is a known molecule used for a very long time for the treatment of cutaneous ageing, acne and Dischromia. Its properties make it extremely efficacious and unique within its kind. Indeed, it has the capacity to regularise sebaceous secretions, thin the corneous layer, thicken the epidermis, organise the distribution of melanin and, above all, by acting through specific receptors, succeeds in reaching the cell nucleus and modulating gene transcription; it is the latter which is its most important characteristic in the therapy of photo-induced cutaneous ageing.

The constant application of retinoic acid to the skin may however result in the onset of an adverse reaction defined in the literature as "retinoid dermatitis" which manifests itself through reddening, burning, itching, exfoliation and also palpebral oedema.

For this reason, tretinoine is used medicinally in the form of creams or lotions, at low concentrations. This means *i.e.* creams of 0.025-0.05 % up to lotions reaching 1% by weight. The use of higher amounts of retinoic acid, up to 5% by weight, has been tried in a lotion with encouraging results with reference to "anti-ageing" activity, but with the adverse result that some treatments had to be interrupted due to the onset of significant irritative phenomena.

### Summary of the invention

The problem which lies at the heart of the present invention is that of providing a retinoic acid based cosmetic composition, which may fulfil its anti-ageing activity without giving rise to side effects such as the irritative effects of the epidermis.

Such problem is resolved by a cosmetic composition in the form of a mask according to claim 12 containing retinoic acid together with cosmetically acceptable excipients, wherein said retinoic acid is comprised in amounts from 1% to 20% by weight with respect to said cosmetic excipients, such as depicted in the enclosed claims.

Preferably, the amount of retinoic acid will be comprised of between 1% and 18% by weight, with respect to the weight of said cosmetic excipients.

More preferably, the amount of retinoic acid will be comprised of between 4% and 16% by weight, still more preferably, it will be greater than 5% and up to 15% by weight, with respect to the weight of the cosmetic excipients.

By the term "retinoic acid" in the present description is meant trans-retinoic acid (natural isomer, all-trans retinoic acid), the various cis isomers of retinoic acid, 3,4-didehydroretinoic acid, or mixtures thereof.

The mask of the invention will preferably be a self-curing mask.

### Detailed description of the invention

The mask of the invention comprises an amount by weight of retinoic acid, calculated according to the weight of the cosmetic excipients, of from 1% to 20%, preferably between 1% and 18%, more preferably between 4% and 16%, still more preferably, greater than 5% and up to 15%.

The mask of the invention allows using high concentrations of active ingredient without having any adverse irritative reactions, and hence significantly reducing the clinical result attainment times. The mask, by virtue of its "occlusive pharmaceutical form" nature, by increasing the bioavailability of the active ingredient, allows the attainment of maximum penetration of retinoic acid, significantly reducing the number of applications and their frequency. For example, if the low concentration cream must be applied twice daily continuously for 3-4 months, conversely the mask may be applied every 7-14 days for 3-5 times. The clinical results are much more evident and the side effects are absent.

Indeed, creams and lotions remain on the cuteous even after treatment, whilst the removal of the self-curing mask allows the reduction of the contact time of the retinoic acid with the skin. The increased bioavailability of the retinoic acid administered in this form allows its rapid penetration as far as reaching the target cells.

Furthermore, the occlusive form traps water within the skin thus inhibiting cutaneous transpiration: water performs an anti-inflammatory action.

This all makes the incidence of the occurrence of retinoid reactions practically insignificant, and hence allows the attainment of the clinical result in rapid times and without any discomfort for the patient.

The mask of the invention can be a facial mask or a mask for neck or hands, or a mask for any part of the body, as the need may be.

The mask of the invention comprises retinoic acid together with cosmetically acceptable excipients. Such excipients will be selected from those normally used for the preparation of masks, particularly for the preparation of self-curing masks.

The amounts of the individual cosmetically acceptable excipients reported throughout the description are calculated depending on the total weight of the excipients in the composition.

Particularly, the composition of the invention will contain a hydrophilic polymer intended to form the load bearing structure of the mask. Such hydrophilic polymer will preferably be selected from vinyl polymers with hydrophilic characteristics. More preferably, the hydrophilic polymer will be polyvinyl alcohol. Such hydrophilic polymer may be used in amounts by weight generally comprised of between 5% and 22%, preferably between 8% and 18%, more preferably between 9% and 13%, calculated based on the total weight of the excipients.

The mask may further contain ethyl alcohol, normally 95° ethyl alcohol, or other alcohol or cosmetically acceptable solvent, functioning as a mask curing time modulating agent. Generally, by increasing the concentration of ethyl alcohol, a reduction in curing time is obtained, and vice versa. Normally, amounts by weight of ethyl alcohol comprised of between 10% and 20% with respect to the total weight of the excipients will be used. With an ethyl alcohol amount of around 15%, mask curing times in the order of 35-40 minutes will be obtained. Longer curing times will be obtained with ethyl alcohol amounts of around 10% by weight. Choice of the curing time will be made in the light of particular clinical exigencies. Indeed, the curing time of the mask also determines the time the formulation spends in contact with the skin, in that only the cured mask may be removed.

The mask of the invention may furthermore contain gelifying agents. The gelifying agents normally used will be selected from acrylic polymers with molecular weight preferably comprised of between 940 and 2001. Preferred acrylic polymers will be high dispersability acrylic polymers, such as the ETD class acrylic polymers. Said acrylic polymers will preferably be used in amounts by weight comprised of between 0.1% and 1.5%, more preferably between 0.2% and 1%, still more preferably between 0.3% and 0.7%. Other usable gelifying agents are celluloses, preferably hydroxyethylcellulose. The celluloses are usable in amounts preferably comprised of between 0.1% and 1%, more preferably between 0.3% and 0.7%. Other usable gelifying agents are selected from among the gums, such as tragacanth gum or xanthan gum, more preferably the latter. The amounts of gum used will preferably vary between 0.1% and 1%, more preferably between 0.3% and 0.7%.

The use of gelifying agents in lesser or greater amounts has the aim of giving the composition a lesser or greater consistency. The use of acrylic polymers is generally preferred to that of celluloses, in that the latter tend to adhere better to the cuteous, thus being able to perform, during the removal of the mask, a depilating effect.

The mask of the invention may furthermore contain pH regulators, particularly acidity regulators. Such additives will preferably be alkaline substances such as cosmetically acceptable organic or inorganic bases. An example of an organic base is triethanolamine. The organic bases will preferably be used in amounts comprised of between 0.1% and 0.6 %, more preferably between 0.2% and 0.4%. An example of an inorganic base is sodium hydroxide in dilute solution, preferably a 1N solution. The inorganic bases will preferably be used in concentrations comprised of between 0.1% and 0.2%.

The mask of the invention may furthermore contain a humectant agent. Such humectant agent will preferably be a polyol, preferably a triol or a glycol. Particularly preferred humectant agents will be glycerine and/or propylene glycol. Preferred amounts of humectant agent will vary between 1% and 20%, preferably between 2% and 17%, more preferably between 4% and 10%, still more preferably between 5% and 8%. Said humectant agents may also be used in mixtures. Particularly, a glycol, preferably propylene glycol, may be used in order to solubilise the retinoic acid during the preparation of the cosmetic composition of the invention, as will be better described in the following.

The mask of the invention may also contain chelating agents or sequestrants. A specific example of a chelating agent is ethylenediaminetetraacetic acid (EDTA). The amount of chelating agent used in the composition of the invention will preferably vary between 0.01% and 0.2%, more preferably between 0.05% and 0.15%.

The mask of the invention may furthermore contain cosmetically acceptable non-ionic surfactants, such as for example PEG 7 glyceryl cocoate, PEG 6 triglyceryl caproic glycerides, polyquaternum 7. Such surfactants, taken individually or in mixture, will preferably be used in variable amounts between 0.5% and 6%, more preferably between 1.5% and 4.5%.

The mask of the invention may furthermore comprise preservatives, preferably in amounts comprised of between 0.1% and 1.5%, more preferably between 0.2% and 1%. Usable preservatives are for example selected from among methyl parabenes and/or imidazolidinyl urea, singularly or in mixtures thereof.

The cosmetic composition of the invention will finally comprise water, in sufficient amount in order to adjust the composition of the excipients to 100%, with a diluent/solubilising function.

The cosmetic composition is made particularly "soft" through the addition of a cream base of the type normally used for the face, the hands or the body. The amount of cream base will preferably vary between 0.2% and 1%, more preferably between 0.4% and 0.7%, still more preferably around 5%, with respect to the weight of the excipients. According to the needs dictated by the typology of the patients skin, it will be possible to use a cream base for normal skin types, for greasy skin types and for mixed skin types.

The composition of such cream base will not differ from the standard creams normally used for such purposes and comprises (% by weight calculated based on the total weight of the cream):
- a lipid component, preferably between 2% and 8%;
- an emollient, preferably between 0.5% and 15%;
- a humectant agent, between 0% and 2%;
- a gelifying agent, between 0% and 1%;
- a surfactant, between 1% and 10%;
- a preservative, between 0.3% and 1%;
- collagen, between 0% and 20%;
- a pH regulator, sufficient to correct the pH to physiological values;
- water, sufficient to adjust the composition of the cream to 100%.

Generally, a higher percentage of lipid component will be indicated in the case of cream for greasy skin types.

High percentages of emollient will be indicated for normal skin types, whilst lower amounts will be used on greasy skin types.

The use of collagen will be particularly indicated for normal skin types.

The cosmetic excipients used in the cream will be generally but not limitingly selected from among those listed previously in the various categories of substances and will be generally selected from among those normally used in analogous cosmetic preparations, well known to any expert in the art.

By way of example, a description will now be provided of the general preparation method of the cosmetic composition of the invention and some examples of specific compositions which are to be used as self-curing masks.

### PREPARATION OF THE COSMETIC COMPOSITION

The preparation of the cosmetic composition in the form of a mask according to the invention defined in claim 43 provides a first operative stage (Stage I), wherein a stock composition of the excipients is obtained, and a subsequent operative stage (Stage II) wherein said stock composition is combined with a solution of retinoic acid. The first stage is in turn divided into two steps A and B.

### STAGE I

### Step A

The chelating agent and/or sequestrant and the gelifying agent are added to the predetermined amount of water. This is allowed to stand for around 2 hours, after which it is neutralised with an acidity regulator and the surfactant is added. In the case of various surfactants, these may be added sequentially or pre-mixed.

### Step B

The hydrophilic polymer and the humectant agent are heated to around 80°C and the resulting mixture is combined with the mixture obtained in step A, itself also heated to around 80°C.

The mixture resulting from the combining of the products of steps A and B is cooled to around 35°C and the preservative agent and the mask curing time modulating agent, normally ethyl alcohol, are added.

### STAGE II

The desired amount of retinoic acid, in the proportions previously indicated, determined according to the clinical needs of the patient, are added to the solvent, preferably propylene glycol, which has been previously heated to around 40°C, after which the resulting solution is blended by turbo-emulsification into the stock composition of Stage I.

The amount of solvent (propylene glycol) used in order to solubilise the retinoic acid generally varies from 0.5% to 10% by weight with respect to the total weight of the excipients.

As mentioned previously, the mask may be made even softer through the addition, in the prior indicated proportions, of a cream base, prepared according to the normal methods well known to any expert in the art, by starting from the raw materials listed above.

### EXAMPLE 1 - Preparation of a cosmetic composition for a self-curing mask

The method described above for the preparation of 100 g of stock composition of excipients is followed. The stock composition of excipients has the following percentage composition (by weight):

| | |
|---|---|
| Carbopol 940 ETD | 0.5 |
| Triethanolamine | 0.3 |
| Purified water | 63.4 |
| Glycerine | 6 |
| Imidazolidinyl urea | 0.3 |
| EDTA | 0,1 |
| Polyvinyl alcohol | 10 |
| Methyl parabenes phenoxyethanol | 0,5 |
| 95° ethyl alcohol | 14.9 |
| Peg 7 glyceryl cocoate | 1.5 |
| Peg 6 Triglyceryl Caproic Glycerides | 1.5 |
| Polyquaternum 7 | 1 |

15 g of retinoic acid is dissolved in 10 g of propylene glycol at 80°C and such solution is added, according to the method previously set out, to the stock composition of excipients.

The resulting composition is inserted into 20 ml capacity aluminium tubes for storage.

### EXAMPLE 2

The preparation of example 1 is repeated, using an amount of retinoic acid of 10 g.

0.5 g of a cream for normal skin types, as defined below, is added to the cosmetic composition.

### EXAMPLE 3

The preparation of example 1 is repeated, using an amount of retinoic acid of 5 g.

0.5 g of a cream for greasy skin types, as defined below, is added to the cosmetic composition.

### EXAMPLE 4

The preparation of example 2 is repeated, but the cream base for normal skin types is substituted with a cream base for mixed skin types.

### PREPARATION 1 - Cream base for normal skin types

2000 g of cream base is obtained according to the normal methods used for the preparation of cosmetic cream, by starting from the following raw materials:

| | |
|---|---|
| Acemulgor LAM V | 160 g |
| Isopropylmyristate | 60 g |
| Tegosoft CT | 80 g |
| Cetylstearyl alcohol | 60 g |
| Glycerine | 20 g |
| Xanthan gum | 6 g |
| Purified water | 1240 g |
| Abil B 8839 | 60 g |
| Sepicide Hb | 8 g |
| Collagen Hyal | 300 g |
| Sepicide CI | 6 g |
| Lactic acid | 100 drops |

### PREPARATION 2 - Cream base for greasy skin types

1,000 g of cream base is obtained according to the normal methods used for the preparation of cosmetic cream, by starting from the following raw materials:

| | |
|---|---|
| PLANTA CREAM V 40 | 100 g |
| ACEMOL OCT | 60 g |
| ISOFOL 20 | 40 g |
| TEGOSOFT CT | 40 g |
| ABIL 350 | 10 g |
| WATER | 740 g |
| PROPYLENE GLYCOL | 5 g |
| SEPICIDE CI | 2 g |
| SEPICIDE HB | 3 g |

### PREPARATION 3 - Cream base for mixed skin types

2000 g of cream base is obtained according to the normal methods used for the preparation of cosmetic cream, by starting from the following raw materials:

| | |
|---|---|
| ACEMULGOR EC | 130 g |
| ACEMOL OCT | 70 g |
| ACEMOL L | 60 g |
| LIGHT VASELINE OIL | 40 g |
| WATER | 1450 g |
| GLYCERINE | 20 g |
| WATER | 200 g |
| SEPICIDE CI | 6 g |
| SEPICIDE HB | 4 g |
| CETYLSTEARYL ALCOHOL | 20 g |

### MASK APPLICATION METHOD

Application of the mask must be carried out by skilled medical staff and in a suitable environment.

The preparation has the appearance of a dense yellow paste which must be spread homogeneously over the body part of the patient to be treated, for example the patient's face, covering all the areas affected by signs of ageing from solar rays and paying close attention to the most sensitive areas, such as the areas closest to the eyes and mouth.

The mask of example 1 cures over a period of approx. 30 minutes, following which it is easily removed by an action known as "tearing off".

The treatment may be repeated every 7-14 days for a number of sessions which varies depending on the skin requiring treatment. Normally the number of sessions will be from 3 to 5.

Absolute contraindications to the treatment are constituted by a known allergy towards retinoic acid and pregnancy.

The skin treatment method of a patient subject to signs of cutaneous ageing according to the invention allows the attainment of excellent results, superior to those obtained with known methods which make use of small amounts of retinoic acid, with the surprising result of not provoking the typical irritation by retinoids which normally already occur with much lower doses of such active ingredient.

## Claims

1. A non-therapeutic skin treatment method for a patient subject to signs of cutaneous ageing, comprising the stages of:
- spreading a cosmetic composition for a self-curing mask, containing retinoic acid together with cosmetically acceptable excipients and a cosmetic cream base with the following percentage composition by weight:
- a lipid component, preferably between 2% and 8%;
- an emollient, preferably between 0.5% and 15%;
- a humectant agents, between 0% and 2%;
- a gasifying agent, between 0% and 1%;
- a surfactant, between 1% and 10%;
- a preservative, between 0.3% and 1%;
- collagen, between 0% and 20%;
- a pH regulator, sufficient to correct the pH to physiological values;
- water, sufficient to adjust the composition of the cream to 100%,
over the body part of said patient subject to signs of cutaneous ageing, wherein said retinoic acid is comprised in amounts of from 1% to 20% by weight with respect to said cosmetic excipients, thus obtaining a mask;
- waiting for the curing of said mask;
- removing said mask by tearing off from the treated part of the body of said patient.

2. The skin treatment method according to claim 1, wherein the treated part of the body of said patient is the face, the hands or the neck.

3. The skin treatment method according to claim 1, wherein said retinoic acid is contained in amounts comprised of between 1% and 18% by weight with respect to the weight of said cosmetic excipients.

4. The skin treatment method according to claim 1, wherein said retinoic acid is contained in amounts comprised of between 4% and 16% by weight with respect to the weight of said cosmetic excipients,

5. The skin treatment method according to claim 1, wherein said retinoic acid is contained in amounts greater than 5% and up to 15% by weight with respect to the weight of said cosmetic excipients.

6. The skin treatment method according to claim 1, wherein said retinoic acid is all-trans-retinoic acid.

7. The skin treatment method according to claim 1, wherein said cosmetic composition comprises a mask curing time modulating agent.

8. The skin treatment method according to claim 1, wherein said cosmetic composition comprises a mask curing time modulating agent, said mask curing time modulating agent being ethyl alcohol.

9. The skin treatment method according to claim 1, wherein said cosmetic composition comprises a mask curing time modulating agent, said mask curing time modulating agent being ethyl alcohol in an amount comprised of between 10% and 20% by weight with respect to the total weight of said excipients.

10. The skin treatment method according to claim 1, wherein the curing time of said mask is comprised of between 30 and 40 minutes.

11. The skin treatment method according to claim 1, said skin treatment being repeated every 7-14 days for a number of sessions of from 3 to 5.

12. The cosmetic composition in the form of a self-curing mask, containing retinoic acid together with cosmetically acceptable excipients and a cosmetic cream base with the following percentage composition by weight:
- a lipid component, preferably between 2% and 8%;
- an emollient, preferably between 0.5% and 15%;
- a humectant agents, between 0% and 2%;
- a gasifying agent, between 0% and 1%;
- a surfactant, between 1% and 10%;
- a preservative, between 0.3% and 1%;
- collagen, between 0% and 20%;
- a pH regulator, sufficient to correct the pH to physiological values;
- water, sufficient to adjust the composition of the cream to 100%,
wherein said retinoic acid is comprised in amounts to from 1% to 20% by weight with respect to said cosmetic excipients, the said mask being apt to be removed by tearing off.

13. The cosmetic composition in the form of a self-curing mask according to claim 12, wherein said retinoic acid is contained in amounts comprised of between 1% and 18% by weight with respect to the weight of said cosmetic excipients.

14. The cosmetic composition in the form of a self-curing mask according to claim 12, wherein said retinoic acid is contained in amounts comprised of between 4% and 16% by weight with respect to the weight of said cosmetic excipients.

15. The cosmetic composition in the form of a self-curing mask according to claim 12, wherein said retinoic acid is contained in amounts greater than 5% and up to 15% by weight with respect to the weight of said cosmetic excipients.

16. The cosmetic composition in the form of a shelf-curing mask according to claim 12, comprising a hydrophilic polymer in amounts by weight comprised of between 5% and 22% with respect to the weight of said cosmetic excipients.

17. The cosmetic composition in the form of a self-curing mask according to claim 12, comprising a hydrophilic polymer in amounts by weight comprised of between 8% and 18% with respect to the weight of said cosmetic excipients.

18. The cosmetic composition in the form of a self-curing mask according to claim 12, comprising a hydrophilic polymer in amounts by weight comprised of between 9% and 13% with respect to the weight of said cosmetic excipients.

19. The cosmetic composition in the form of a self-curing mask according to claim 12, comprising a hydrophilic polymer selected from among vinyl polymers with hydrophilic characteristics such as polyvinyl alcohol.

20. The cosmetic composition in the form of a self-curing mask according to claim 12, comprising a hydrophilic polymer selected from among vinyl polymers with hydrophilic characteristics, said hydrophilic polymer being polyvinyl alcohol in amounts by weight comprised of between 9% and 13% calculated on the basis of the total weight of the excipients.

21. The cosmetic composition in the form of a self-curing mask according to claim 12, comprising a mask curing time modulating agent, said modulating agent being ethyl alcohol in amounts by weight comprised of between 10% and 20% with respect to the total weight of the excipients.

22. The cosmetic composition in the form of a self-curing mask according to claim 12, comprising gelifying agents.

23. The cosmetic composition in the form of a self-curing mask according to claim 12, comprising gelifying agents selected from among acrylic polymers.

24. The cosmetic composition in the form of a self-curing mask according to claim 12, comprising gelifying agents selected from among high dispersability ETD class acrylic polymers.

25. The cosmetic composition in the form of a self-curing mask according to claim 12, comprising gelifying agents selected from among acrylic polymers in amounts by weight comprised of between 0.1% and 1.5% with respect to the weight of the cosmetic excipients.

26. The cosmetic composition in the form of a shelf - curing mask according to claim 12, comprising gelifying agents selected from among the celluloses, such as hydroxyethylcellulose, in amounts by weight comprised of between 0.1% and 1% with respect to the weight of the cosmetic excipients.

27. The cosmetic composition in the form of a self-curing mask according to claim 12, comprising gelifying agents selected from among the gums, such as tragacanth gum or xanthan gum, in amounts by weight comprised of between 0.1% and 1% with respect to the weight of the cosmetic excipients.

28. The cosmetic composition in the form of a self-curing mask according to claim 12, comprising pH regulators.

29. The cosmetic composition in the form of a self-curing mask according to claim 12, comprising acidity regulators selected from among cosmetically acceptable organic or inorganic bases.

30. The cosmetic composition in the form of a self-curing mask according to claim 12, comprising triethanolamine in amounts by weight comprised of between 0.1% and 0.6 % with respect to the weight of the cosmetic excipients.

31. The cosmetic composition in the form of a self-curing mask according to claim 12, comprising a 1 N solution of sodium hydroxide in amounts by weight comprised of between 0.1% and 0.2% with respect to the weight of the cosmetic excipients.

32. The cosmetic composition in the form of a self-curing mask according to claim 12, comprising a humectant agent.

33. The cosmetic composition in the form of a self-curing mask according to claim 12, comprising a humectant agent selected from a polyol or a glycol, such as glycerine and/or propylene glycol.

34. The cosmetic composition in the form of a self-curing mask according to claim 12, comprising a humectant agent in amounts by weight comprised of between 1% and 20%.

35. The cosmetic composition in the form of a self-curing mask according to claim 12, comprising chelating agents or sequestrants.

36. The cosmetic composition in the form of a self-curing mask according to claim 12, comprising chelating agents or sequestrants such as ethylenediaminetetraacetic acid, in an amount by weight comprised of between 0.01% and 0.2% with respect to the weight of the cosmetic excipients.

37. The cosmetic composition in the form of a self-curing mask according to claim 12, comprising cosmetically compatible non-ionic surfactants.

38. The cosmetic composition in the form of a self-curing mask according to claim 12, comprising cosmetically compatible non-ionic surfactants such as PEG 7 glyceryl cocoate, PEG 6 triglyceryl caproic glycerides or polyquaternum 7, in amounts by weight comprised of between 0.5% and 6% with respect to the weight of the cosmetic excipients.

39. The cosmetic composition in the form of a self-curing mask according to claim 12, comprising preservatives.

40. The cosmetic composition in the form of a self-curing mask according to claim 12, comprising preservatives, such as methyl parabenes and/or imidazolidinyl urea, in amounts by weight comprised of between 0.1% and 1.5% with respect to the weight of the cosmetic excipients.

41. The cosmetic composition in the form of a self-curing mask according to claim 12, said composition being an aqueous composition.

42. The cosmetic composition in the form of a self-curing mask according to claim 12, wherein the said cosmetic cream base is in an amount by weight comprised of between 0.2% and 1% with respect to the weight of the excipients.

43. A preparation process for a cosmetic composition in the form of a self-curing mask, comprising the following operative stages:
a) preparing an aqueous mixture of chelating agent and/or sequestrant, gelifying agent, acidity regulator and surfactant;
b) preparing a mixture of hydrophilic polymer and humectant agent;
c) combining the two resulting mixtures from steps a) and b) ;
d) combining the resulting mixture from step c) with the preservative agent and the mask curing time modulating agent;
e) incorporating a solution of retinoic acid in amount of from 1% to 20% by weight with respect to said cosmetic excipients in a solvent into said mixture obtained from step d).

44. The process according to claim 43, wherein the mixtures from steps a) and b) are heated to around 80°C prior to being combined.

45. The process according to claim 43, wherein said retinoic acid is dissolved in said solvent at a temperature of around 40°C.

46. The process according to claim 43, wherein said dissolution solvent for the retinoic acid is propylene glycol in an amount comprised of between 0.5% and 10% by weight with respect to the total weight of the other excipients of the composition.

## Patentansprüche

1. Nichttherapeutisches Hautbehandlungsverfahren für einen Patienten, der Anzeichen einer Hautalterung zeigt, umfassend die Schritte:
- Verteilen einer kosmetischen Zusammensetzung für eine selbsthärtende Maske, enthaltend Retinolsäure, zusammen mit kosmetisch verträglichen Exzipienten und eine kosmetische Cremebasis mit der folgenden prozentualen Zusammensetzung bezüglich des Gewichts:
- eine Lipidkomponente, vorzugsweise zwischen 2% und 8%;
- ein erweichendes Mittel, vorzugsweise zwischen 0,5% und 15%;
- ein Feuchthaltemittel, zwischen 0% und 2%;
- ein Geliermittel, zwischen 0% und 1%;
- ein oberflächenaktives Mittel, zwischen 1 % und 10%;
- ein Konservierungsmittel, zwischen 0,3% und 1 %
- Collagen, zwischen 0% und 20%;
- ein pH-Wert-Regulationsmittel, ausreichend, um den
pH-Wert auf physiologische Werte zu korrigieren;
- Wasser, ausreichend, um die Zusammensetzung der Creme auf 100% zu bringen,
über das Körperteil des Patienten, das Anzeichen einer Hautalterung zeigt, worin die Retinolsäure in Mengen von 1 Gew.-% bis 20 Gew.-% bezüglich der kosmetischen Exzipienten enthalten ist, wobei eine Maske erhalten wird;
- Abwarten des Aushärtens der Maske;
- Entfernen der Maske durch Abziehen von dem behandelten Teil des Körpers des Patienten.

2. Hautbehandlungsverfahren nach Anspruch 1, worin der behandelte Teil des Körpers des Patienten das Gesicht, die Hände oder der Hals ist.

3. Hautbehandlungsverfahren nach Anspruch 1, worin die Retinolsäure in Mengen enthalten ist, umfassend von zwischen 1 Gew.-% und 18 Gew.-% bezüglich des Gewichts der kosmetischen Exzipienten.

4. Hautbehandlungsverfahren nach Anspruch 1, worin die Retinolsäure in Mengen enthalten ist, umfassend von zwischen 4 Gew.-% und 16 Gew.-% bezüglich des Gewichts der kosmetischen Exzipienten.

5. Hautbehandlungsverfahren nach Anspruch 1, worin die Retinolsäure in Mengen enthalten ist, die größer als 5 Gew.-% und bis zu 15 Gew.-% bezüglich des Gewichts der kosmetischen Exzipienten sind.

6. Hautbehandlungsverfahren nach Anspruch 1, worin die Retinolsäure all-trans-Retinolsäure ist.

7. Hautbehandlungsverfahren nach Anspruch 1, worin die kosmetische Zusammensetzung ein Maskeaushärtungszeit-Modulierungsmittel umfasst.

8. Hautbehandlungsverfahren nach Anspruch 1, worin die kosmetische Zusammensetzung ein Maskeaushärtungszeit-Modulierungsmittel umfasst, wobei das Maskeaushärtungszeit-Modulierungsmittel Ethylalkohol ist.

9. Hautbehandlungsverfahren nach Anspruch 1, worin die kosmetische Zusammensetzung ein Maskeaushärtungszeit-Modulierungsmittel umfasst, wobei das Maskeaushärtungszeit-Modulierungsmittel, das Ethylalkohol ist, in einer Menge vorliegt, umfassend von zwischen 10 Gew.-% und 20 Gew.-% bezüglich des Gesamtgewichts der Exzipienten.

10. Hautbehandlungsverfahren nach Anspruch 1, worin die Aushärtungszeit der Maske zwischen 30 und 40 Minuten umfasst.

11. Hautbehandlungsverfahren nach Anspruch 1, worin die Hautbehandlung alle 7-14 Tage für 3 bis 5 Sitzungen wiederholt wird.

12. Kosmetische Zusammensetzung in Form einer selbsthärtenden Maske, enthaltend Retinolsäure zusammen mit kosmetisch verträglichen Exzipienten und eine kosmetische Cremebasis mit der folgenden prozentualen Zusammensetzung bezüglich des Gewichts:
- eine Lipidkomponente, vorzugsweise zwischen 2% und 8%;
- ein erweichendes Mittel, vorzugsweise zwischen 0,5% und 15%;
- ein Feuchthaltemittel, zwischen 0% und 2%;
- ein Geliermittel, zwischen 0% und 1%;
- ein oberflächenaktives Mittel, zwischen 1% und 10%;
- ein Konservierungsmittel, zwischen 0,3% und 1 %
- Collagen, zwischen 0% und 20%;
- ein pH-Wert-Regulationsmittel, ausreichend, um den pH-Wert auf physiologische Werte zu korrigieren;
- Wasser, ausreichend, um die Zusammensetzung der Creme auf 100% einzustellen, worin die Retinolsäure in Mengen von 1 Gew.-% bis 20 Gew.-% bezüglich der kosmetisch verträglichen Exzipienten enthalten ist, wobei die Maske durch Abziehen zu entfernen ist.

13. Kosmetische Zusammensetzung in Form einer selbsthärtenden Maske nach Anspruch 12, worin die Retinolsäure in Mengen, umfassend von zwischen 1 Gew.-% und 18 Gew.-% bezüglich des Gewichts der kosmetischen Exzipienten enthalten ist.

14. Kosmetische Zusammensetzung in Form einer selbsthärtenden Maske nach Anspruch 12, worin die Retinolsäure in Mengen, umfassend von zwischen 4 Gew.-% und 16 Gew.-% bezüglich des Gewichts der kosmetischen Exzipienten enthalten ist.

15. Kosmetische Zusammensetzung in Form einer selbsthärtenden Maske nach Anspruch 12, worin die Retinolsäure in Mengen, umfassend von größer als 5 Gew.-% und bis zu 15 Gew.-% bezüglich des Gewichts der kosmetischen Exzipienten enthalten ist.

16. Kosmetische Zusammensetzung in Form einer selbsthärtenden Maske nach Anspruch 12, umfassend ein hydrophiles Polymer in Mengen bezüglich des Gewichts, umfassend zwischen 5% und 22% bezüglich des Gewichts der kosmetischen Exzipienten.

17. Kosmetische Zusammensetzung in Form einer selbsthärtenden Maske nach Anspruch 12, umfassend ein hydrophiles Polymer in Mengen bezüglich des Gewichts, umfassend zwischen 8% und 18% bezüglich des Gewichts der kosmetischen Exzipienten.

18. Kosmetische Zusammensetzung in Form einer selbsthärtenden Maske nach Anspruch 12, umfassend ein hydrophiles Polymer in Mengen bezüglich des Gewichts, umfassend zwischen 9% und 13% bezüglich des Gewichts der kosmetischen Exzipienten.

19. Kosmetische Zusammensetzung in Form einer selbsthärtenden Maske nach Anspruch 12, umfassend ein hydrophiles Polymer, ausgewählt aus Vinylpolymeren mit hydrophilen Charakteristika, wie etwa Polyvinylalkohol.

20. Kosmetische Zusammensetzung in Form einer selbsthärtenden Maske nach Anspruch 12, umfassend ein hydrophiles Polymer, ausgewählt aus Vinylpolymeren mit hydrophilen Charakteristika, wobei das hydrophile Polymer Polyvinylalkohol ist, in Mengen bezüglich des Gewichts, umfassend zwischen 9% und 13%, berechnet auf Basis des Gesamtgewichts der Exzipienten.

21. Kosmetische Zusammensetzung in Form einer selbsthärtenden Maske nach Anspruch 12, umfassend ein Maskeaushärtungszeit-Modulierungsmittel, wobei das Modulierungsmittel, das Ethylalkohol ist, in einer Menge vorliegt, umfassend von zwischen 10 Gew.-% und 20 Gew.-% bezüglich des Gesamtgewichts der Exzipienten.

22. Kosmetische Zusammensetzung in Form einer selbsthärtenden Maske nach Anspruch 12, umfassend Geliermittel.

23. Kosmetische Zusammensetzung in Form einer selbsthärtenden Maske nach Anspruch 12, umfassend Geliermittel, ausgewählt aus Acrylpolymeren.

24. Kosmetische Zusammensetzung in Form einer selbsthärtenden Maske nach Anspruch 12, umfassend Geliermittel, ausgewählt aus Acrylpolymeren mit hoher Dispergierbarkeit der ETD-Klasse.

25. Kosmetische Zusammensetzung in Form einer selbsthärtenden Maske nach Anspruch 12, umfassend Geliermittel ausgewählt aus Acrylpolymeren, in Mengen bezüglich des Gewichts, umfassend von zwischen 0,1% und 1,5% bezüglich des Gewichts der kosmetischen Exzipienten.

26. Kosmetische Zusammensetzung in Form einer selbsthärtenden Maske nach Anspruch 12, umfassend Geliermittel, ausgewählt aus den Cellulosen, wie etwa Hydroxyethylcellulose, in Mengen bezüglich des Gewichts , umfassend zwischen 0,1% und 1 % bezüglich des Gewichts der kosmetischen Exzipienten.

27. Kosmetische Zusammensetzung in Form einer selbsthärtenden Maske nach Anspruch 12, umfassend Geliermittel, ausgewählt aus den Gummis, wie etwa Tragantgummi oder Xanthangummi, in Mengen bezüglich des Gewichts, umfassend zwischen 0,1% und 1% bezüglich des Gewichts der kosmetischen Exzipienten.

28. Kosmetische Zusammensetzung in der Form einer selbsthärtenden Maske nach Anspruch 12, umfassend pH-Wert-Regulationsmittel.

29. Kosmetische Zusammensetzung in der Form einer selbsthärtenden Maske nach Anspruch 12, umfassend Acidität-Regulationsmittel, ausgewählt aus kosmetisch verträglichen organischen oder anorganischen Basen.

30. Kosmetische Zusammensetzung in der Form einer selbsthärtenden Maske nach Anspruch 12, umfassend Triethanolamin, in Mengen bezüglich des Gewichts, umfassend zwischen 0,1% und 0,6% bezüglich des Gewichts der kosmetischen Exzipienten.

31. Kosmetische Zusammensetzung in der Form einer selbsthärtenden Maske nach Anspruch 12, umfassend eine 1 N-Lösung von Natriumhydroxid, in Mengen bezüglich des Gewichts, umfassend zwischen 0,1% und 0,2% bezüglich des Gewichts der kosmetischen Exzipienten.

32. Kosmetische Zusammensetzung in der Form einer selbsthärtenden Maske nach Anspruch 12, umfassend ein Feuchthaltemittel.

33. Kosmetische Zusammensetzung in der Form einer selbsthärtenden Maske nach Anspruch 12, umfassend ein Feuchthaltemittel, ausgewählt aus einem Polyol oder einem Glykol, wie etwa Glycerin und/oder Propylenglykol.

34. Kosmetische Zusammensetzung in der Form einer selbsthärtenden Maske nach Anspruch 12, umfassend ein Feuchthaltemittel, in Mengen bezüglich des Gewichts, umfassend zwischen 1 % und 20%.

35. Kosmetische Zusammensetzung in der Form einer selbsthärtenden Maske nach Anspruch 12, umfassend Chelatisierungsmittel oder Maskierungsmittel.

36. Kosmetische Zusammensetzung in der Form einer selbsthärtenden Maske nach Anspruch 12, umfassend Chelatisierungsmittel oder Maskierungsmittel, wie etwa Ethylendiamintetraessigsäure, in einer Menge bezüglich des Gewichts, umfassend von zwischen 0,01 % und 0,2% bezüglich des Gewichts der kosmetischen Exzipienten.

37. Kosmetische Zusammensetzung in der Form einer selbsthärtenden Maske nach Anspruch 12, umfassend kosmetisch verträgliche nichtionische oberflächenaktive Mittel.

38. Kosmetische Zusammensetzung in der Form einer selbsthärtenden Maske nach Anspruch 12, umfassend kosmetisch verträgliche nichtionische oberflächenaktive Mittel wie etwa PEG 7-Glyceryl-kakaoat, PEG 6-Triglyceryl-capronglyceride oder Polyquaternum 7, in Mengen bezüglich des Gewichts, umfassend von zwischen 0,5% und 6% bezüglich des Gewichts der kosmetischen Exzipienten.

39. Kosmetische Zusammensetzung in der Form einer selbsthärtenden Maske nach Anspruch 12, umfassend Konservierungsmittel.

40. Kosmetische Zusammensetzung in der Form einer selbsthärtenden Maske nach Anspruch 12, umfassend Konservierungsmittel, wie etwa Methylparabene und oder Imidazolidinylharnstoff, in Mengen bezüglich des Gewichts, umfassend von zwischen 0,1% und 1,5% bezüglich des Gewichts der kosmetischen Exzipienten.

41. Kosmetische Zusammensetzung in der Form einer selbsthärtenden Maske nach Anspruch 12, worin die Zusammensetzung eine wässrige Zusammensetzung ist.

42. Kosmetische Zusammensetzung in der Form einer selbsthärtenden Maske nach Anspruch 12,worin die kosmetische Cremebasis in einer Menge bezüglich des Gewichts vorliegt, umfassend von zwischen 0,2% und 1% bezüglich des Gewichts der kosmetischen Exzipienten.

43. Herstellungsverfahren für eine kosmetische Zusammensetzung in der Form einer selbsthärtenden Maske, umfassend die folgenden Arbeitsschritte:
a) Herstellen eines wässrigen Gemischs von Chelatisierungsmittel und/oder Maskierungsmittel, Geliermittel, Aciditäts-Regulationsmittel und oberflächenaktivem Mittel;
b) Herstellen eines Gemischs von hydrophilem Polymer und Feuchthaltemittel;
c) Vereinigen der beiden resultierenden Gemische aus den Schritten a) und b);
d) Vereinigen des aus Schritt c) resultierenden Gemischs mit dem Konservierungsmittel und dem Maskeaushärtungszeit-Modulierungsmittel;
e) Einbringen einer Lösung von Retinolsäure in einer Menge von 1 % bis 20% bezüglich des Gewichts, bezogen auf die kosmetischen Exzipienten, in einem Lösungsmittel in das aus Schritt d) erhaltene Gemisch..

44. Verfahren nach Anspruch 43, worin die Gemische aus den Schritten a) und b) auf etwa 80°C erhitzt werden bevor sie vereinigt werden.

45. Verfahren nach Anspruch 43, worin die Retinolsäure in dem Lösungsmittel bei einer Temperatur von etwa 40°C gelöst wird.

46. Verfahren nach Anspruch 43, worin das Lösungmittel für die Retinolsäure Propylenglykol ist, in einer Menge, umfassend zwischen 0,5% und 10% bezüglich des Gewichts, bezogen auf das Gesamtgewicht der anderen Exzipienten der Zusammensetzung.

## Revendications

1. Procédé de traitement de la peau non thérapeutique pour un patient sujet à des signes de vieillissement cutané, comprenant les étapes de :
- étalement d'une composition cosmétique pour un masque auto-durcissant, contenant de l'acide rétinoïque, des excipients cosmétiquement acceptables et une base de crème cosmétique avec le pourcentage de composition en poids suivant :
- un composant lipidique, de préférence entre 2 et 8 % ;
- un émollient, de préférence entre 0,5 et 15 % ;
- un agent humectant, entre 0 et 2 % ;
- un agent gélifiant, entre 0 et 1% ;
- un tensioactif, entre 1 et 10 % ;
- un conservateur, entre 0,3 et 1 %.
- du collagène, entre 0 et 20 % ;
- un régulateur de pH, suffisant pour corriger le pH à des valeurs physiologiques ;
- de l'eau, suffisamment pour ajuster la composition de la crème à 100 %
sur la partie du corps dudit patient sujet à des signes de vieillissement cutané, dans lequel ledit acide rétinoïque est compris en quantités de 1 à 20 % en poids par rapport auxdits excipients cosmétiques, donnant ainsi un masque ;
- attente du durcissement dudit masque ;
- enlèvement dudit masque par le détachement de la partie traitée du corps dudit patient.

2. Procédé de traitement de la peau selon la revendication 1, dans lequel la partie traitée du corps dudit patient est le visage, les mains ou le cou.

3. Procédé de traitement de la peau selon la revendication 1, dans lequel ledit acide rétinoïque est contenu en quantités comprises entre 1 et 18 % en poids par rapport au poids desdits excipients cosmétiques.

4. Procédé de traitement de la peau selon la revendication 1, dans lequel ledit acide rétinoïque est contenu en quantités comprises entre 4 et 16 % en poids par rapport au poids desdits excipients cosmétiques.

5. Procédé de traitement de la peau selon la revendication 1, dans lequel ledit acide rétinoïque est contenu en quantités supérieures à 5 % et jusqu'à 15 % en poids par rapport au poids desdits excipients cosmétiques.

6. Procédé de traitement de la peau selon la revendication 1, dans lequel ledit acide rétinoïque est l'acide tout trans-rétinoïque.

7. Procédé de traitement de la peau selon la revendication 1, dans lequel ladite composition cosmétique comprend un agent de modulation du temps de durcissement du masque.

8. Procédé de traitement de la peau selon la revendication 1, dans lequel ladite composition cosmétique comprend un agent de modulation du temps de durcissement du masque, ledit agent de modulation du temps de durcissement du masque étant l'alcool éthylique.

9. Procédé de traitement de la peau selon la revendication 1, dans lequel ladite composition cosmétique comprend un agent de modulation du temps de durcissement du masque, ledit agent de modulation du temps du durcissement du masque étant l'alcool éthylique en une quantité comprise entre 10 et 20 % en poids par rapport au poids total desdits excipients.

10. Procédé de traitement de la peau selon la revendication 1, dans lequel le temps de durcissement dudit masque est compris entre 30 et 40 minutes.

11. Procédé de traitement de la peau selon la revendication 1, ledit traitement de la peau étant répété tous les 7 à 14 jours pour un nombre de sessions de 3 à 5.

12. Composition cosmétique sous la forme d'un masque auto-durcissant, contenant de l'acide rétinoïque, des excipients cosmétiquement acceptables et une base de crème cosmétique avec le pourcentage de composition en poids suivant :
- un composant lipidique, de préférence entre 2 et 8 % ;
- un émollient, de préférence entre 0,5 et 15 % ;
- un agent humectant, entre 0 et 2 % ;
- un agent gélifiant, entre 0 et 1 % ;
- un tensioactif, entre 1 et 10 % ;
- un conservateur, entre 0,3 et 1 %.
- du collagène, entre 0 et 20 % ;
- un régulateur de pH, suffisant pour corriger le pH à des valeurs physiologiques ;
- de l'eau, suffisamment pour ajuster la composition de la crème à 100 %
, dans laquelle ledit acide rétinoïque est compris en quantités de 1 à 20 % en poids par rapport audits excipients cosmétiques, ledit masque pouvant être enlevé par détachement.

13. Composition cosmétique sous la forme d'un masque auto-durcissant selon la revendication 12, dans laquelle ledit acide rétinoïque est contenu en quantités comprises entre 1 et 18 % en poids par rapport au poids desdits excipients cosmétiques.

14. Composition cosmétique sous la forme d'un masque auto-durcissant selon la revendication 12, dans laquelle ledit acide rétinoïque est contenu en quantités comprises entre 4 et 16 % en poids par rapport au poids desdits excipients cosmétiques.

15. Composition cosmétique sous la forme d'un masque auto-durcissant selon la revendication 12, dans laquelle ledit acide rétinoïque est contenu en quantités supérieures à 5 % et jusqu'à 15 % en poids par rapport au poids desdits excipients cosmétiques.

16. Composition cosmétique sous la forme d'un masque auto-durcissant selon la revendication 12, comprenant un polymère hydrophile en quantités en poids comprises entre 5 et 22 % par rapport au poids desdits excipients cosmétiques.

17. Composition cosmétique sous la forme d'un masque auto-durcissant selon la revendication 12, comprenant un polymère hydrophile en quantités en poids comprises entre 8 et 18 % par rapport au poids desdits excipients cosmétiques.

18. Composition cosmétique sous la forme d'un masque auto-durcissant selon la revendication 12, comprenant un polymère hydrophile en quantités en poids comprises entre 9 et 13 % par rapport au poids desdits excipients cosmétiques.

19. Composition cosmétique sous la forme d'un masque auto-durcissant selon la revendication 12, comprenant un polymère hydrophile choisi parmi les polymères vinyliques avec des caractéristiques hydrophiles tels que l'alcool polyvinylique.

20. Composition cosmétique sous la forme d'un masque auto-durcissant selon la revendication 12, comprenant un polymère hydrophile choisi parmi les polymères vinyliques avec des caractéristiques hydrophiles, ledit polymère hydrophile étant l'alcool polyvinylique en quantités en poids comprises entre 9 et 13 % calculées par rapport au poids total des excipients.

21. Composition cosmétique sous la forme d'un masque auto-durcissant selon la revendication 12, comprenant un agent de modulation du temps de durcissement du masque, ledit agent de modulation étant l'alcool éthylique en quantités en poids comprises entre 10 et 20 % par rapport au poids total des excipients.

22. Composition cosmétique sous la forme d'un masque auto-durcissant selon la revendication 12, comprenant des agents gélifiants.

23. Composition cosmétique sous la forme d'un masque auto-durcissant selon la revendication 12, comprenant des agents gélifiants choisis parmi les polymères acryliques.

24. Composition cosmétique sous la forme d'un masque auto-durcissant selon la revendication 12, comprenant des agents gélifiants choisis parmi les polymères acryliques de classe ETD à dispersibilité élevée.

25. Composition cosmétique sous la forme d'un masque auto-durcissant selon la revendication 12, comprenant des agents gélifiants choisis parmi les polymères acryliques en quantités en poids comprises entre 0,1 et 1,5 % par rapport au poids des excipients cosmétiques.

26. Composition cosmétique sous la forme d'un masque auto-durcissant selon la revendication 12, comprenant des agents gélifiants choisis parmi les celluloses, telles que l'hydroxyéthylcellulose, en quantités en poids comprises entre 0,1 et 1 % par rapport au poids des excipients cosmétiques.

27. Composition cosmétique sous la forme d'un masque auto-durcissant selon la revendication 12, comprenant des agents gélifiants choisis parmi les gommes, telles que la gomme adragante ou la gomme de xanthane, en quantités en poids comprises entre 0,1 et 1 % par rapport au poids des excipients cosmétiques.

28. Composition cosmétique sous la forme d'un masque auto-durcissant selon la revendication 12, comprenant des régulateurs de pH.

29. Composition cosmétique sous la forme d'un masque auto-durcissant selon la revendication 12, comprenant des régulateurs d'acidité choisis parmi les bases organiques ou minérales cosmétiquement acceptables.

30. Composition cosmétique sous la forme d'un masque auto-durcissant selon la revendication 12, comprenant de la triéthanolamine en quantités en poids comprises entre 0,1 et 0,6 % par rapport au poids des excipients cosmétiques.

31. Composition cosmétique sous la forme d'un masque auto-durcissant selon la revendication 12, comprenant une solution 1 N d'hydroxyde de sodium en quantités en poids comprises entre 0,1 et 0,2 % par rapport au poids des excipients cosmétiques.

32. Composition cosmétique sous la forme d'un masque auto-durcissant selon la revendication 12, comprenant un agent humectant.

33. Composition cosmétique sous la forme d'un masque auto-durcissant selon la revendication 12, comprenant un agent humectant choisi parmi un polyol ou un glycol, tel que la glycérine et/ou le propylène glycol.

34. Composition cosmétique sous la forme d'un masque auto-durcissant selon la revendication 12, comprenant un agent humectant en quantités en poids comprises entre 1 et 20 %.

35. Composition cosmétique sous la forme d'un masque auto-durcissant selon la revendication 12, comprenant des agents chélatants ou des agents séquestrants.

36. Composition cosmétique sous la forme d'un masque auto-durcissant selon la revendication 12, comprenant des agents chélatants ou des agents séquestrants tels que l'acide éthylènediaminotétraacétique, en quantités en poids comprises entre 0,01 et 0,2 % par rapport au poids des excipients cosmétiques.

37. Composition cosmétique sous la forme d'un masque auto-durcissant selon la revendication 12, comprenant des tensioactifs non ioniques cosmétiquement compatibles.

38. Composition cosmétique sous la forme d'un masque auto-durcissant selon la revendication 12, comprenant des tensioactifs non ioniques cosmétiquement compatibles tels que le PEG 7 cocoate de glycéryle, les PEG 6 glycérides caproïques de triglycéryle ou le polyquatemum 7, en quantités en poids comprises entre 0,5 et 6 % par rapport au poids des excipients cosmétiques.

39. Composition cosmétique sous la forme d'un masque auto-durcissant selon la revendication 12, comprenant des conservateurs.

40. Composition cosmétique sous la forme d'un masque auto-durcissant selon la revendication 12, comprenant des conservateurs, tels que les méthyl parabènes et/ou l'imidazolidinyle urée, en quantités en poids comprises entre 0,1 et 1,5 % par rapport au poids des excipients cosmétiques.

41. Composition cosmétique sous la forme d'un masque auto-durcissant selon la revendication 12, ladite composition étant une composition aqueuse.

42. Composition cosmétique sous la forme d'un masque auto-durcissant selon la revendication 12, dans laquelle ladite base de crème cosmétique est en une quantité en poids comprise entre 0,2 et 1 % par rapport au poids des excipients

43. Procédé de préparation pour une composition cosmétique sous la forme d'un masque auto-durcissant, comprenant les étapes opératoires suivantes :
a) préparation d'un mélange aqueux d'un agent chélatant et/ou d'un agent séquestrant, d'un agent gélifiant, d'un régulateur d'acidité et d'un tensioactif ;
b) préparation d'un mélange d'un polymère hydrophile et d'un agent humectant ;
c) combinaison des deux mélanges obtenus aux étapes a) et b) ;
d) combinaison du mélange obtenu à l'étape c) avec l'agent conservateur et l'agent de modulation du temps de durcissement du masque ;
e) incorporation d'une solution d'acide rétinoïque en une quantité de 1 à 20 % en poids par rapport audit excipient cosmétique dans un solvant dans ledit mélange obtenu à l'étape d).

44. Procédé selon la revendication 43, dans lequel les mélanges des étapes a) et b) sont chauffés à environ 80°C avant d'être combinés.

45. Procédé selon la revendication 43, dans lequel ledit acide rétinoïque est dissous dans ledit solvant à une température d'environ 40°C.

46. Procédé selon la revendication 43, dans lequel ledit solvant de dissolution pour l'acide rétinoïque est le propylène glycol en une quantité comprise entre 0,5 et 10 % en poids par rapport au poids total des autres excipients de la composition.
